# EUROPÄISCHE PATENTSCHRIFT

(11) **EP 0 410 263 B1**
(45) Veröffentlichungstag und Bekanntmachung des Hinweises auf die Patenterteilung: **16.02.1994**
(21) Anmeldenummer: 90113601.0
(22) Anmeldetag: 16.07.1990
(51) Int. Cl.: C07C 381/12, C08F 2/50, G03F 7/029

(54) **Verfahren zur photoinitiierten kationischen Polymerisation sowie zur Herstellung von Reliefmustern oder Reliefbildern unter Verwendung von Sulfoniumsalzen**
Cationic photoinitiated polymerisation process and the preparation of relief motifs or photoimages using sulfonium salts
Procédé de polymérisation cationique photoinitiée et de préparation de motifs et de photographies en relief à l'aide de sels de sulfonium

(30) Priorität: 22.07.1989 DE 3924298
(43) Veröffentlichungstag der Anmeldung: 30.01.1991
(73) Patentinhaber: BASF Aktiengesellschaft, 67063 Ludwigshafen (DE)
(72) Erfinder: Schwalm, Reinhold, Dr., D-6706 Wachenheim (DE)

(56) Entgegenhaltungen:
- EP-A- 0 331 988
- EP-A- 0 346 756
- DE-A- 3 721 740
- PEPTIDE CHEMISTRY, 1987, Seiten 435-438, Protein Research Foundation, Osaka, JP; Y. KIMURA et al.: "A useful enzyme for removing fatty acyl groups from N-acyl proteins, peptides, amino acids, and amino carbohydrates"
- BULLETIN OF THE CHEMICAL SOCIETY OF JAPAN, Band 60, 1987, Seiten 4343-4349, The Chemical Society of Japan, Tokyo, JP; K. KOUGE et al.: "Peptide synthesis in aqueous solution. II. Synthesis and biological activity of a molluscan neuropeptide, FMRFamide (Phe-Met-Arg-Phe-NH2) analogs for N-terminal moiety"
- CHEMICAL ABSTRACTS, Band 111, 2. Oktober 1989, Seite 503, Zusammenfassung Nr. 122749g, Columbus, Ohio, US; & JP-A-01 83 679
- CHEMICAL ABSTRACTS SERVICE - NUMBER SECTION, 1989 supplement, Registry Numbers 122517-10-6 bis 124535-32-6, Seite 3937 RR, Absatz 122931-43-5, 122931-44-6, 122931-45-7; & CHEMICAL ABSTRACTS, Band 111, 1989, Seite 783, Zusammenfassung Nr. 154383z, 154384a, Columbus, Ohio, US; & JP-A-01 50 897, & JP-A-01 50 896
- JOURNAL OF AGRICULTURAL AND FOOD CHEMISTRY, Band 38, Januar 1990, Seiten 25-29, Washington, US; T. SEKI et al.: "Further study on the salty peptide ornithyl-beta-alanine. Some effects of pH and additive ions on the saltiness"
- JOURNAL OF AGRICULTURAL AND FOOD CHEMISTRY, Band 38, Juni 1990, Seiten 1368-1373, American Chemical Society, Washington, US; Y. NOSHO et al.: "Molecular design of inverted-aspartame-type sweeteners"
- AGRICULTURAL AND BIOLOGICAL CHEMISTRY, Band 53, Juni 1989, Seiten 1625-1633, Tokyo, JP; M. TAMURA et al.: "An enhancing effect on the saltiness of sodium chloride of added amino acids and their esters"
- CHEMICAL ABSTRACTS, Band 112, 14. Mai 1990, Seite 150, Zusammenfassung Nr. 181909c, Columbus, Ohio, US; & JP-A-01 85 125
- CHEMICAL ABSTRACTS, Band 112, 18. Juni 1990, Seite 591, Zusammenfassung Nr. 234992h, Columbus, Ohio, US; & JP-A-01 290 658

## Beschreibung

Die Erfindung betrifft ein Verfahren zur photoinitiierten kationischen Polymerisation sowie zur Herstellung von Reliefmustern oder Reliefbildern unter Verwendung von Sulfoniumsalzen, die zusätzlich zur Sulfoniumgruppierung bestimmte funktionale Gruppen enthalten. Diese Sulfoniumsalze erzeugen unter der Einwirkung von Strahlung eine Säure, die dann Sekundärreaktionen ermöglicht bzw. katalysieren kann. Das Löslichkeitsverhalten der Verbindungen läßt sich drastisch ändern, indem die erzeugte Säure oder ein nachfolgender Behandlungsschritt die Polarität der Sulfoniumsalze vollständig verändert. Die neuen Sulfoniumsalze eignen sich besonders als Photoinitiatoren für die kationische Polymerisation sowie als Initiatoren in Photoresisten.

Sulfoniumsalze sind seit langem in der Literatur bekannt (vgl. z.B. H.M. Pitt, US-A-2 807 648 (1957); W. Hahn und R. Stroh, US-A-2 833 827 (1958)>; G.H. Wiegand und W.E. McEwen, J. Org. Chem., 33, 2671 (1968)).

Als Photoinitiatoren kommen fast ausschließlich Sulfoniumsalze mit komplexen, nichtnucleophilen Gegenionen in Frage, wie die von Crivello entwickelten Photoinitiatoren für die kationische Polymerisation (vgl. z.B. US-A-4 058 400 und US-A-4 058 401). Einen Überblick über die Verwendung von Oniumsalzen bei der kationischen Polymerisation gibt Crivello in "Cationic Polymerisation - Iodonium and Sulfonium Salt Photoinitiators", Advances in Polym. Sci., 62, 1-48 (1984).

Die Verwendung von Oniumsalzen in Photoresistmaterialien ist z.B. in "Possibilities for Photoimaging Using Onium Salts", Crivello in Corporate Research and Development, General Electric, Schenectady, New York (1983) sowie von Ito und Willson in Org. Ctgs. and App. Polym. Sci. Proc. 48, 60 (1983) und US-A-4 491 628 beschrieben.

Photochemisch säurebildende Sulfoniumsalze haben sich in der kationischen Polymerisation sowie in der Photoresisttechnik bewährt. Die Modifikation an den Sulfoniumsalzen beschränkten sich in der Vergangenheit im wesentlichen darauf, die Löslichkeit mit Hilfe von inerten Substituenten und das Absorptionsverhalten an die Hauptemissionslinien der verwendeten Lichtquellen anzupassen. Die in DE-A 37 21 740 beschriebenen Sulfoniumsalze mit säurelabilen Gruppen und nichtnucleophilen Gegenionen enthalten neben der säurebildenden Sulfoniumgruppierung noch andere funktionale Gruppen.

Die bisher beschriebenen Sulfoniumsalze sind sehr effektive Initiatoren für die Polymerisation bzw. effektive Säurespender in Photoresistmaterialien. Als Löslichkeitsinhibitoren in alkalilöslichen Matrices sind diese einfachen Salze jedoch nur bedingt geeignet. Zum Beispiel beschreibt H. Ito in SPIE Vol. 920 Advances in Resist Technology and Processing V (1988), Seite 35, daß diese Salze nicht in der Lage sind die Löslichkeit von Poly-(p-hydroxystyrol) effektiv zu inhibieren. In DE-A-37 21 740 werden Oniumsalze beschrieben, die neben der säurebildenden Oniumgruppierung noch säurelabile Gruppen enthalten. Diese Salze kann man als Löslichkeitsinhibitoren in alkalilösliche Matrices, auch für Poly-(p-hydroxystyrol) einsetzen, jedoch benötigt man recht hohe Konzentrationen an Sulfoniumsalzen.

Aufgabe der Erfindung war es, ein Verfahren zur photoinitiierten kationischen Polymerisation sowie zur Herstellung von Reliefmustern und Reliefbildern unter Verwendung von Sulfoniumsalzen aufzuzeigen, um so das Konzept der Verbindung von säurebildenden Oniumgruppen und anderen funktionalen, nach der Belichtung bzw. weiteren Behandlungsschritten wieder abspaltbaren, Gruppen in einem Molekül, zu erweitern.

Es wurde nun gefunden, daß man mit speziellen Sulfoniumsalzen, die neben der Sulfoniumgruppe zusätzlich im gleichen Molekül bestimmte funktionale Gruppen enthalten, die gestellte Aufgabe hervorragend lösen kann.

Gegenstand der vorliegenden Erfindung ist ein Verfahren zur photoinitiierten kationischen Polymerisation bzw. zur Herstellung von Reliefmustern und Reliefbildern, das dadurch gekennzeichnet ist, daß mindestens ein Sulfoniumsalz der allgemeinen Formel (I) 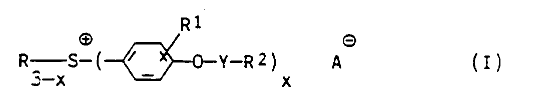
worin
- x =: 1, 2 oder 3,
- R =: Alkyl, Cycloalkyl, Aryl, substituiertes Aryl oder für den Fall x = 1 ein divalenter cyclischer Rest mit S^{⊕} als Ringglied,
- R¹ =: H, Alkyl, Alkoxy, Halogen oder Nitrogruppe,
- R² =: mehr als 6 Kohlenstoffatome enthaltender einwertiger aliphatischer oder aromatischer Rest, der gegebenenfalls ein oder mehrere Heteroatome enthält, ein alicyclischer oder durch Alkyl, Alkoxy, -O-CHO, -O-CO-CH₃ oder OH substituierter alicyclischer Rest oder der Rest eines Photosensibilisatormoleküls,
- Y =: Einfachbindung, 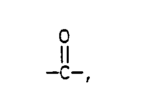 -SO₂-, 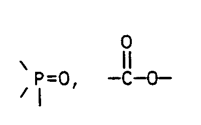 oder 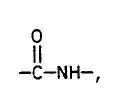
- A^{⊖} =: nichtnucleophiles Gegenion.
als Photoinitiator bzw. Löslichkeitsinhibitor eingesetzt wird.

Bevorzugte Ausführungsformen der erfindungsgemäßen Sulfoniumsalze bestehen darin, daß in der allgemeinen Formel (I) R² für einen alicyclischen oder für einen durch Alkyl, Alkoxy, -O-CHO, -O-CO-CH₃ oder OH substituierten alicyclischen Rest steht, R² für den Rest eines Photosensibilisatormoleküls steht, R² für einen geradkettigen oder verzweigten aliphatischen Kohlenwasserstoffrest mit mindestens 12 Kohlenstoffatomen oder einen Fluorkohlenwasserstoffrest mit mindestens 8 Kohlenstoffatomen, oder R² für einen basenlabilen Rest steht.

Gegenstand der vorliegenden Erfindung ist also die Verwendung der obengenannten Sulfoniumsalze zur photoinitiierten kationischen Polymerisation, sowie zur Herstellung von Reliefmustern oder Reliefbildern, wobei mindestens eines dieser Sulfoniumsalze als Photoinitiator und Löslichkeitsinhibitor eingesetzt wird.

Die erfindungsgemäßen Sulfoniumsalze sind sensitiv gegenüber UV-, Elektronen- und Röntgenstrahlen. Als Gegenionen eignen sich besonders nichtnucleophile Anionen, z.B. komplexe Metallhalogenide, wie Hexafluoroantimonat, Hexafluoroarsenat oder Tetrafluoroborat sowie Trifluoromethansulfonat, p-Toluolsulfonat, Perchlorat oder Fluorsulfonat.

Die erfindungsgemäß einzusetzenden Sulfoniumsalze mit nichtnucleophilen Gegenionen erzeugen bei Einwirkung von Strahlung eine starke Säure, die weitere Sekundärreaktionen, wie Initiieren einer kationischen Polymerisation, Initiieren einer säurekatalysierten Vernetzung oder Depolymerisation, ermöglicht. Die alkaliunlöslichen Salze können dann entweder durch Einwirkung der gebildeten Säure oder durch Behandlung mit Basen in basenlösliche phenolische Verbindungen umgewandelt werden.

Erfindungsgemäß einzusetzende Sulfoniumsalze sind also beispielsweise solche, die neben der Sulfoniumgruppe mindestens eine umgesetzte phenolische Gruppe im Molekül enthalten, so daß die funktionalen Gruppen über Ether, Ester, Carbonat oder Urethanbindungen im Molekül enthalten sind, beispielsweise solche der allgemeinen Formel (I) 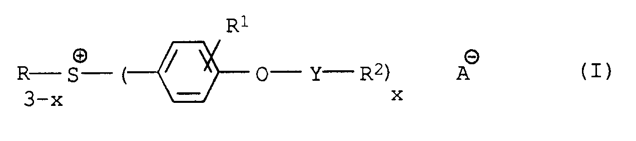
worin
- x =: 1, 2 oder 3,
- R =: Alkyl, z.B. mit 1 bis 6, vorzugsweise 1 bis 4 Kohlenstoffatomen, wie z.B. Methyl, Ethyl, Butyl, Cycloalkyl, wie z.B. Cyclohexyl, Aryl, wie z.B. Phenyl, substituiertes Aryl, wie z.B. t-Butylphenyl oder für den Fall x = 1 ein divalenter cyclischer Rest mit S^{⊕} als Ringglied, wie z.B. ein Tetrahydrothiophenring,
- R¹ =: H, Alkyl, z.B. mit 1 bis 6 Kohlenstoffatomen, wie z.B. Methyl, Ethyl, Alkoxy, z.B. mit 1 bis 6 Kohlenstoffatomen, wie z.B. Methoxy, Butoxy, Halogen, wie z.B. Chlor, Fluor, oder Nitrogruppe,
- R² =: mehr als 6 Kohlenstoffatome enthaltender einwertiger aliphatischer oder aromatischer Rest, der gegebenenfalls ein oder mehrere Heteroatome, wie z.B. N, S und/oder O, enthält,
- Y =: Einfachbindung, 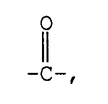 -SO₂-, 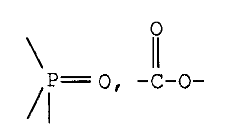 oder 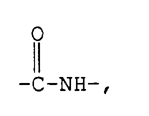 vorzugsweise 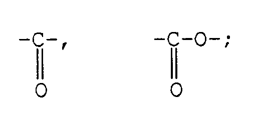
- A^{⊖} =: nichtnucleophiles Gegenion, wie z.B. Hexafluoroantimonat, Hexafluoroarsenat, Tetrafluoroborat, Trifluoromethansuflonat, p-Toluolsulfonat, Perchlorat oder Fluorosulfonat.

### Beispiele für derartige Sulfoniumsalze sind:

1. Sulfoniumsalze mit sterisch anspruchsvollen Gruppen, die über säurelabile Gruppierungen an das Molekül gebunden sind und als effektive Löslichkeitsinhibitoren wirken, wobei in der allgemeinen Formel (I) Y für den Rest 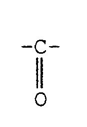 oder 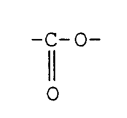 und R² für einen alicyclischen oder substituierten alicyclischen Rest, d.h. für einen sterisch anspruchsvollen aliphatischen Rest steht, wie z.B. den 1-Adamantanrest, den Noradamantanrest oder die Reste 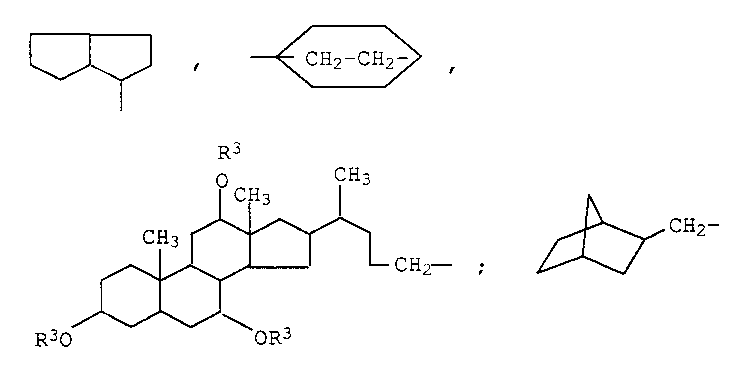
   - R³ =: H, CHO, -CO-CH₃
2. Sulfoniumsalze, enthaltend Reste von Photosensibilisatormolekülen, wie z.B. 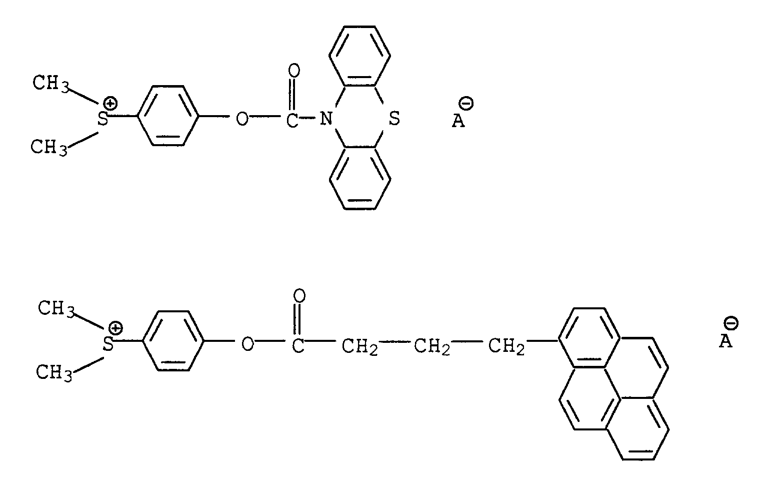
3. Sulfoniumsalze, enthaltend als R² in der allgemeinen Formel (I) einen geradkettigen oder verzweigten aliphatischen Kohlenwasserstoffrest mit mindestens 12, vorzugsweise 12 bis 30 Kohlenstoffatomen oder einen Fluorkohlenwasserstoff mit mindestens 8, vorzugsweise 8 bis 18 Kohlenstoffatomen, also längerkettige hydrophobe Reste, die oberflächenaktive Wirkung zeigen: 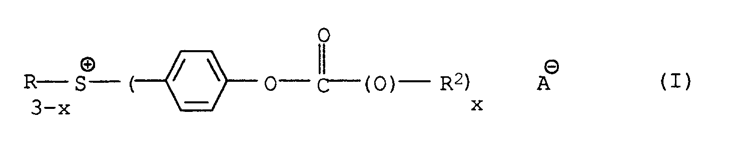
   - R² =: aliphatische KW-Reste mit C ≧ 12,-(CH₂)ₙ-CH₃ mit n ≧ 11 = aliphatische Fluorkohlenwasserstoffreste mit C ≧ 8,

   -(CR'₂)ₙ-CR'₃

   n = ≧ 7; R' = H und/oder F.
4. Sulfoniumsalze, enthaltend basenlabile Gruppierungen, wie z.B. 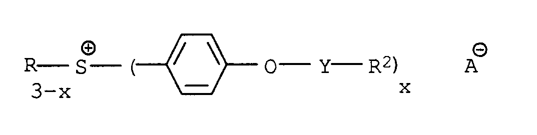 mit 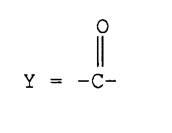 und 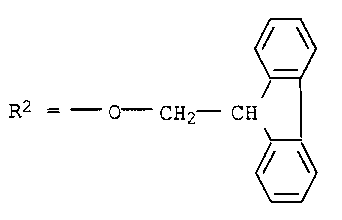 (fluorenyl-methyl-oxi)

In den erfindungsgemäß einzusetzenden Sulfoniumsalzen der Formel 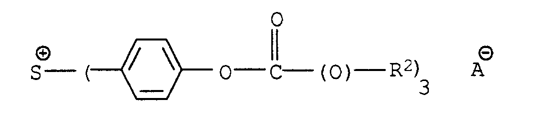
sind also, wie oben bereits erwähnt, als Gruppen R² bevorzugt aliphatische, sterisch anspruchsvolle Reste, wie der Adamantanrest, Nordadamantanrest, Cholsäurerest, Norbornylessigsäurerest oder Cholesterylrest. Diese Reste sind bevorzugt über Ester oder Carbonatgruppen am Molekül gebunden, so daß nach der Photoreaktion und säurekatalysierter Hydrolyse alkalilösliche phenolische Photoprodukte entstehen, etwa nach dem Schema 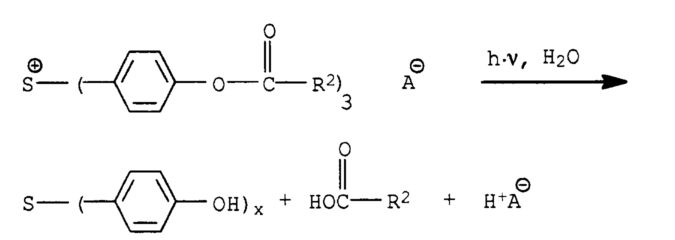
und andere
Für manche Anwendungen, z.B. wenn man die Absorptionscharakteristik der Sulfoniumsalze nicht verändern will, jedoch eine leichte Auswaschbarkeit in Alkali benötigt, ist es günstig, wenn man säurestabile Schutzgruppen am Sulfoniumsalz hat, welche sich erst bei der Entwicklung mit Alkali in phenolische Produkte und somit leicht lösliche, spalten. Daher gehören zu den erfindungsgemäß einzusetzenden Sulfoniumsalzen auch Sulfoniumsalze mit basenlabilen, jedoch säurestabilen Schutzgruppen. Bevorzugt sind beispielsweise solche Sulfoniumsalze der Formel 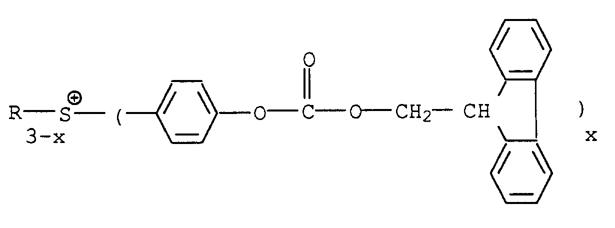
worin die Fälle x = 1 R = CH₃; sowie x = 3 und R fehlt, bevorzugt sind.

Weiterhin sind für manche Anwendungen Verbindungen von Vorteil, die sich bevorzugt an der Oberfläche anlagern. Beispiele dazu sind die in "Macromolecules 1985, 18, 1804" vorgeschlagenen Polymerisationen von Monomeren an einer den (kationischen) Initiator enthaltenden Grenzfläche. Es hat sich gezeigt, daß solche Sulfoniumsalze, die lange aliphatische Ketten enthalten, bevorzugt an der Grenzfläche Substrat/Luft angelagert werden, wenn man sie in polare Substrate einbringt. Erfindungsgemäß bevorzugt sind daher auch Sulfoniumsalze mit Kohlenwasserstoffresten mit C ≧ 12 Kohlenstoffatomen bzw. mit Fluorkohlenwasserstoffresten R² mit C ≧ 8 Kohlenstoffatomen, beispielsweise 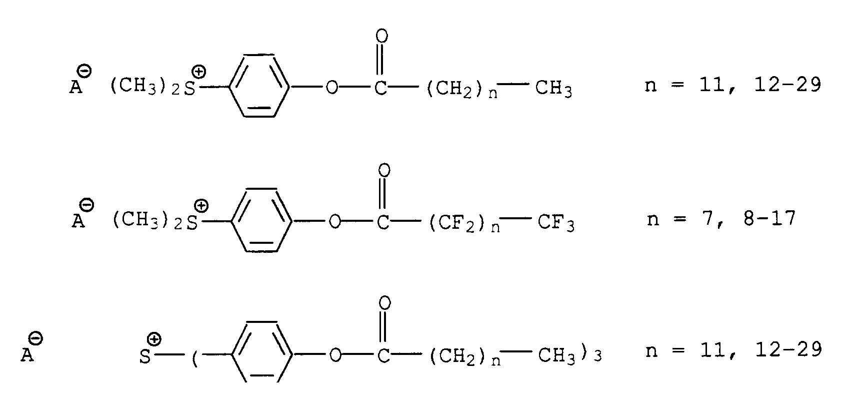
Beispiele für erfindungsgemäß einzusetzende Sulfoniumsalze, die einen Sensibilisator im Molekül gebunden haben, wurden bereits oben erwähnt. In "Polymer Photochemistry 5, 1-22 (1984)" ist beschrieben, daß sich Iodonium- und Sulfoniumsalze sensibilisieren lassen. Besonders geeignet sind kondensierte Aromaten, wie Perylen, Anthracen und Phenothiazin. Durch Ankoppeln der Sensibilisatorgruppen an dem zu sensibilisierenden Molekülteil kann man die Effektivität der Sensibilisierung steigern. Erfindungsgemäß werden Sulfoniumsalze folgender Formel bevorzugt: 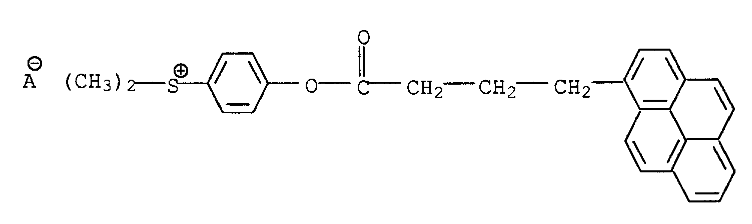
Die erfindungsgemäß einzusetzenden Sulfoniumsalze können nach den bekannten Methoden der organischen Chemie zur Synthese von Estern, Carbonaten, Ethern und Urethanen hergestellt werden, indem man von bekannten Sulfoniumsalzen mit phenolischen Gruppen ausgeht und diese so umsetzt, daß die funktionalen Gruppen über Ester, Carbonate, Ether oder Urethane an den Phenolrest gebunden sind.

Die hierzu verwendbaren Ausgangsverbindungen sind bekannt.

Hydroxyphenyl-dialkyl-sulfoniumsalze, die bereits ein nichtnucleophiles Gegenion enthalten, können beispielsweise nach einer Synthesevorschrift in J. Polym. Sci., Chem. Ed., 18, 1021 (1980) hergestellt werden.

Bis-(hydroxyphenyl)-aryl-sulfoniumsalze kann man beispielsweise nach der Methode von Crivello in J. Polym. Sci., Ed. 18, 2697 (1980) herstellen, indem man Diaryliodoniumsalze mit nichtnucleophilen Gegenionen mit z.B. Bis-(hydroxyphenyl)-sulfiden unter Kupfer(II)-Katalyse umsetzt. Diese Verbindungen sind auch schon in DE-A-37 21 740 beschrieben.

Tris-(hydroxyphenyl)-sulfoniumsalze lassen sich beispielsweise nach einer Synthesevorschrift aus US-A-2 833 827 herstellen.

Zur Synthese der erfindungsgemäß einzusetzenden Sulfoniumsalze mit funktionalen Gruppen kann folgendes bemerkt werden:
Hydroxyphenyl-bis-alkyl/aryl-, Bis-(hydroxyphenyl)-aryl- bzw. Tris-(hydroxyphenyl)-sulfoniumsalze werden zweckmäßigerweise in indifferenten Lösungsmitteln, wie Acetonitril, Tetrahydrofuran oder Ethylacetat mit Säurechloriden, Chlorameisensäureestern oder Isocyanaten, gegebenenfalls in Gegenwart einer Base, umgesetzt. Die Reaktionsmischungen werden dann in Wasser gegeben und die Sulfoniumsalze können mit beispielsweise Ethylacetat extrahiert, getrocknet und aus einem geeigneten Lösungsmittel umkristallisiert werden.

Die oben genannten Sulfoniumsalze eignen sich als Photoinitiatoren für die photoinitiierte kationische Polymerisation von z.B. Styrol, Alkylvinylethern, cyclischen Ethern und Epoxiden.

Die oben genannten Sulfoniumsalze eignen sich auch sehr vorteilhaft als Photoinitiatoren und Löslichkeitsinhibitoren bei der Herstellung von Photoresistmaterialien, wobei als polymere Bindemittel bevorzugt phenolische Harze, wie Novolake oder Poly-p-hydroxystyrol in Frage kommen.

Die in den Beispielen genannten Teile und Prozente beziehen, soweit nicht anders angegeben, auf das Gewicht.

### Beispiel 1

### Synthese von Tris-[4-(1-adamantancarbonyloxy)phenyl]-sulfoniumtrifluormethansulfonat

Tris-(4-hydroxyphenyl)-sulfoniumchlorid wird nach einer Methode in Chemische Berichte 72, Seite 890 ff. (1939) hergestellt. 3,47 Teile dieses Salzes werden in 20 Teilen Methanol gelöst und mit 2,57 Teilen Silbertrifluormethansulfonat in 10 Teilen Methanol versetzt. Ausgefallenes Silberchlorid wird abgesaugt und die Lösung in einem Rotationsverdampfer eingedampft. Das so erhaltene Rohprodukt wurde ungereinigt für die weiteren Umsetzungen eingesetzt.

| Elementaranalyse: | | | | | | |
|---|---|---|---|---|---|---|
| | C | H | S | F | Cl | Ag |
| gef.: | 49,6 | 3,8 | 13,1 | 11,5 | - | - |
| theor.: | 49,6 | 3,3 | 13,9 | 12,4 | | |

9,2 Teile des so hergestellten Tris-(hydroxyphenyl)-sulfoniumtrifluormethansulfonats werden in 150 Teilen Acetonitril gelöst, 8 Teile Pyridin zugegeben und 11,9 Teile 1-Adamantancarbonsäurechlorid in 50 Teilen Acetonitril langsam zugetropft. Nach Stehen über Nacht filtriert man, dampft das Lösungsmittel ab und kristallisiert aus Tetrahydrofuran/Ligroin um. Ausbeute: 15 Teile des erwarteten Produktes.

IR- und N-NMR Spektren zeigen, daß die phenolische Bande verschwunden und statt dessen eine Esterbande entstanden ist.

| Elementaranalyse: | | | | | |
|---|---|---|---|---|---|
| | C | H | S | F | Cl |
| gef.: | 64,3 | 6,4 | 6,0 | 5,8 | - |
| theor.: | 65,9 | 6.1 | 6,8 | 6,0 | |

### Beispiel 2

### Synthese von Tris-[4-(fluorenylmethyl-oxycarbonyloxy)phenyl]-sulfoniumtrifluormethansulfonat

9,2 Teile des gemäß Beispiel 1 hergestellten Tris-(hydroxyphenyl)-sulfoniumtrifluormethansulfonat werden in 200 Teilen Acetonitril gelöst. Zu dieser Lösung gibt man 8 Teile Pyridin. Dazu tropft man 15,5 Teile Chlorameisensäure(fluorenylmethyl)ester gelöst in 35 Teilen Toluol langsam zu. Danach erhitzt man zwei Stunden am Rückfluß, gibt nach dem Abkühlen 500 Teile 2 %ige Salzsäure zu und extrahiert portionsweise mit 2000 Teilen Ethylacetat. Nach dem Waschen der organischen Lösung wird das Lösungsmittel entfernt. Es resultieren 16 Teile des gewünschten Produkts. IR- und NMR spektroskopische Untersuchungen stimmen mit der erwarteten Struktur überein.

Die basenlabile Schutzgruppe läßt sich durch Behandlung mit 2N NaOH quantitativ entfernen. Die Reaktion läßt sich mit Hilfe der IR und H-NMR Spektroskopie verfolgen.

### Beispiel 3

### Synthese von Dimethyl-4-(pentadecan-carbonyloxy)phenylsulfoniumhexafluoroarsenat

3,44 Teile Dimethyl-(hydroxyphenyl)-sulfoniumhexafluoroarsenat werden in 30 Teilen Aetonitril gelöst und mit 1,01 Teilen Triethylamin versetzt. Dazu tropft man bei Raumtemperatur langsam 2,75 Teile Hexadecansäurechlorid, gelöst in 10 Teilen Acetonitril, erwärmt anschließend zwei Stunden auf dem Wasserbad, filtriert und zieht das Lösungsmittel im Vakuum ab. Die Ausbeute an Sulfoniumsalz beträgt 3,5 Teile. IR und H-NMR spektroskopische Untersuchungen bestätigen die erwartete Struktur.

Die bevorzugte Anlagerung der Sulfoniumsalze an der Oberfläche konnte gezeigt werden, indem 10 % des Salzes in eine Matrix aus einem handelsüblichen Novolak gegeben und in einer Schichtdicke von 1,5 µm auf einen Siliziumwafer aufgeschleudert wurden und anschließend an der Oberfläche mit ESCA-Spektroskopie die Konzentration an Schwefel und Arsen im Vergleich zu einer analog vorbereiteten Probe mit dem nicht umgesetzten Dimethyl-(hydroxyphenyl)-sulfonium-hexafluoroarsenat als Salz bestimmt wurde. Es zeigte sich, daß die Arsen und Schwefelkonzentration im Fall des Sulfoniumsalzes mit langkettigem aliphatischen Rest mehr als doppelt so hoch wie mit dem nicht substituierten Derivat sind.

### Beispiel 4

### Synthese von Dimethyl-4-(3-(1-pyrenyl)propyl-carbonyl)phenylhexafluoroarsenat

Kommerziell erhältliche 4-(1-Pyrenyl)-buttersäure wird mit Thionylchlorid zum Carbonsäurechlorid umgesetzt. 3,06 Teile dieses Chlorids werden dann zu einer Lösung von 3,44 Teilen Dimethyl-(hydroxyphenyl)sulfoniumhexafluoroarsenat in 30 Teilen Acetonitril und 1,01 Teilen Triethylamin gegeben. Man läßt über Nacht stehen, filtriert und entfernt das Lösungsmittel. IR und H-NMR spektroskopische Untersuchungen bestätigen das Vorliegen der erwarteten Verbindung.

### Verwendung als Photoinitiator:

Eine Mischung aus 30 Teilen destilliertem Styrol, 0,5 Teilen des gemäß Beispiel 4 hergestellten Sulfoniumsalzes und 10 Teilen Tetrachlormethan wird unter Stickstoff 15 Minuten mit einer Quecksilber-Lampe (mit Interferenzfilter, transparent bei 365 nm) belichtet. Es findet eine exotherme Polymerisation statt und die Reaktionslösung wird viskos. Danach wird die Reaktionslösung verdünnt und in Methanol gefällt.

## Patentansprüche

1. Verfahren zur photoinitiierten kationischen Polymerisation, dadurch gekennzeichnet, daß als Photoinitiator ein Sulfoniumsalz der allgemeinen Formel (I) 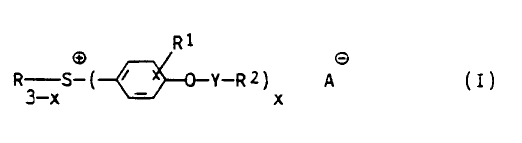 worin
x = 1, 2 oder 3,
R = Alkyl, Cycloalkyl, Aryl, substituiertes Aryl oder für den Fall x = 1 ein divalenter cyclischer Rest mit S^{⊕} als Ringglied,
R¹ = H, Alkyl, Alkoxy, Halogen oder Nitrogruppe,
R² = mehr als 6 Kohlenstoffatome enthaltencer einwertiger aliphatischer oder aromatischer Rest, der gegebenenfalls ein oder mehrere Heteroatome enthält, ein alicyclischer oder durch Alkyl, Alkoxy, -O-CHO, -O-CO-CH₃ oder OH substituierter alicyclischer Rest oder der Rest eines Photosensibilisatormoleküls,
Y = Einfachbindung, 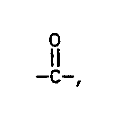 -SO₂-, 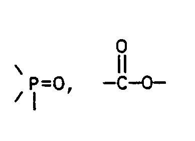 oder 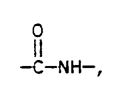
A^{⊖} = nichtnucleophiles Gegenion.
eingesetzt wird.

2. Verfahren nach Anspruch 1, dadurch gekennzeichnet, daß in der allgemeinen Formel (I) R² für einen geradkettigen oder verzweigten aliphatischen Kohlenwasserstoffrest mit mindestens 12 Kohlenstoffatomen oder einen Fluorkohlenwasserstoffrest mit mindestens 8 Kohlenstoffatomen steht.

3. Verfahren nach Anspruch 1, dadurch gekennzeichnet, daß in der allgemeinen Formel (I) R² für einen basenlabilen Rest steht.

4. Verfahren zur Herstellung von Reliefmustern oder Reliefbildern, dadurch gekennzeichnet, daß mindestens ein Sulfoniumsalz der allgemeinen Formel (I) 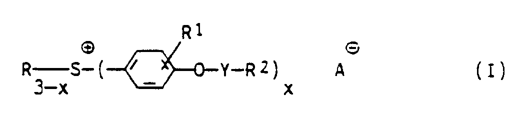 worin
x = 1, 2 oder 3,
R = Alkyl, Cycloalkyl, Aryl, substituiertes Aryl oder für den Fall x = 1 ein divalenter cyclischer Rest mit S^{⊕} als Ringglied,
R¹ = H, Alkyl, Alkoxy, Halogen oder Nitrogruppe,
R² = mehr als 6 Kohlenstoffatome enthaltencer einwertiger aliphatischer oder aromatischer Rest, der gegebenenfalls ein oder mehrere Heteroatome enthält, ein alicyclischer oder durch Alkyl, Alkoxy, -O-CHO, -O-CO-CH₃ oder OH substituierter alicyclischer Rest oder der Rest eines Photosensibilisatormoleküls,
Y = Einfachbindung, 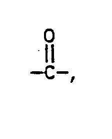 -SO₂-, 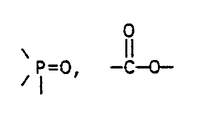 oder 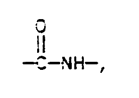
A^{⊖} = nichtnucleophiles Gegenion,
als Photoinitiator und Löslichkeitsinhibitor eingesetzt wird.

5. Verfahren nach Anspruch 4, dadurch gekennzeichnet, daß in der allgemeinen Formel (I) R² für einen geradkettigen oder verzweigten aliphatischen Kohlenwasserstoffrest mit mindestens 12 Kohlenstoffatomen oder einen Fluorkohlenwasserstoff mit mindestens 8 Kohlenstoffatomen steht.

6. Verfahren nach Anspruch 4, dadurch gekennzeichnet, daß in der allgemeinen Formel (I) R² für einen basenlabilen Rest steht.

## Claims

1. A process for photoinitiated cationic polymerization, which comprises using photoinitiator that is a sulfonium salt of the general formula (I) 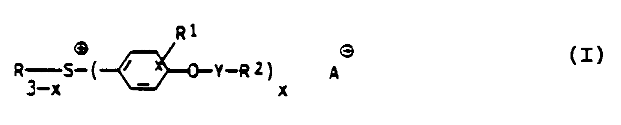 where
x is 1, 2 or 3,
R is alkyl, cycloalkyl, aryl, substituted aryl or - if x is 1 - a divalent cyclic radical containing S^{⊕} as ring member,
R¹ is hydrogen, alkyl, alkoxy, halogen or nitro,
R² is a monovalent aliphatic or aromatic radical of more than 6 carbon atoms which may contain one or more heteroatoms, an unsubstituted alicyclic radical or an alicyclic radical which is substituted by alkyl, alkoxy, -O-CHO, -O-CO-CH₃ or OH or the radical of a photosensitizer molecule,
Y is a single bond 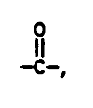 -SO₂-, 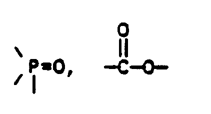 or 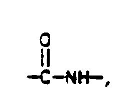 and
A^{⊖} is a non-nucleophilic counterion.

2. A process as claimed in claim 1, wherein in the general formula (I) R² is a straight-chain or branched aliphatic hydrocarbon radical of 12 or more carbon atoms or a fluorocarbon radical of 8 or more carbon atoms.

3. A process as claimed in claim 1, wherein in the general formula (I) R² is a base-labile radical.

4. A process for producing relief patterns or images, which comprises using at least one sulfonium salt of the general formula (I) 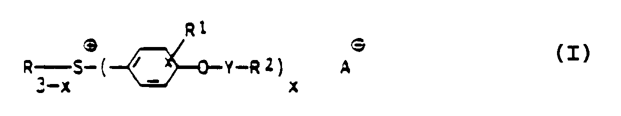 where
x is 1, 2 or 3,
R is alkyl, cycloalkyl, aryl, substituted aryl or - if x is 1 - a divalent cyclic radical containing S^{⊕} as ring member,
R¹ is hydrogen, alkyl, alkoxy, halogen or nitro,
R² is a monovalent aliphatic or aromatic radical of more than 6 carbon atoms which may contain one or more heteroatoms, an unsubstituted alicyclic radical or an alicyclic radical which is substituted by alkyl, alkoxy, -O-CHO, -O-CO-CH₃ or OH or the radical of a photosensitizer molecule,
Y is a single bond 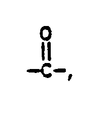 -SO₂-, 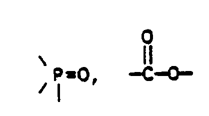 or 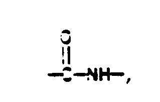 and
A^{⊖} is a non-nucleophilic counterion,
as photoinitiator and solubility inhibitor.

5. A process as claimed in claim 4, wherein in the general formula (I) R² is a straight-chain or branched aliphatic hydrocarbon radical of 12 or more carbon atoms or a fluorocarbon radical of 8 or more carbon atoms.

6. A process as claimed in claim 4, wherein in the general formula (I) R² is a base-labile radical.

## Revendications

1. Procédé de polymérisation cationique photoinitiée caractérisé par le fait que l'on utilise comme photoinitiateur un sel de sulfonium de la formule générale I 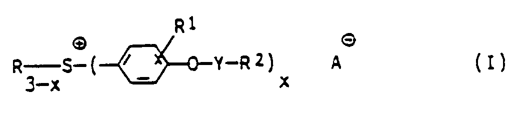 dans laquelle
x = 1, 2 ou 3,
R = alkyle, cycloalkyle, aryle, aryle substitué ou, dans le cas où x = 1, un reste divalent cyclique avec S^{⊕} comme chaînon de cycle,
R¹ = H, alkyle, alcoxy, halogène ou groupe nitro,
R² = reste aliphatique ou aromatique, monovalent, contenant plus de 6 atomes de carbone, et qui contient éventuellement un ou plusieurs hétéroatomes, un reste alicyclique ou un reste alicyclique substitué par alkyle, alcoxy, -O-CHO, -O-CO-CH₃ ou OH, ou le reste d'une molécule de photosensibilisateur,
Y = liaison simple 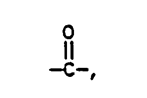 -SO₂-, 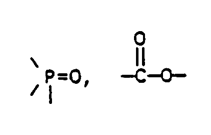 ou 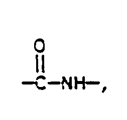 A^{⊖} = ion complémentaire non nucléophile

2. Procédé selon la revendication 1, caractérisé par le fait que, dans la formule générale I, R² est mis pour un reste d'hydrocarbure aliphatique à chaîne droite ou ramifiée ayant au moins 12 atomes de carbone ou un reste d'hydrocarbure fluoré ayant au moins 8 atomes de carbone.

3. Procédé selon la revendication 1, caractérisé par le fait que dans la formule générale I, R² est mis pour un reste instable aux bases.

4. Procédé de préparation de modèles de réserve ou d'images de réserve, caractérisé par le fait que l'on utilise comme photoinitiateur ou inhibiteur de solubilité au moins un sel de sulfonium de la formule générale I 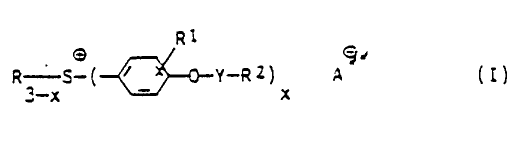 dans laquelle
x = 1,2 ou 3,
R = alkyle, cycloalkyle, aryle, aryle substitué ou, dans le cas où x = 1, un reste divalent cyclique avec S^{⊕} comme chaînon de cycle,
R¹ = H, alkyle, alcoxy, halogène ou groupe nitro,
R² = reste aliphatique ou aromatique, monovalent, contenant plus de 6 atomes de carbone, et qui contient éventuellement un ou plusieurs hétéroatomes, un reste alicyclique ou un reste alicyclique substitué par alkyle, alcoxy, -O-CHO, -O-CO-CH₃ ou OH, ou le reste d'une molécule de photosensibilisateur,
Y = liaison simple 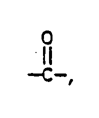 -SO₂-, 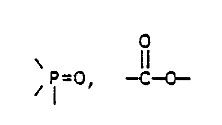 ou 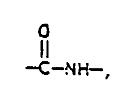 A^{⊖} = ion complémentaire non nucléophile

5. Procédé selon la revendication 4 caractérisé par le fait que dans la formule générale I, R² est mis pour un reste d'hydrocarbure aliphatique à chaîne droite ou ramifiée ayant au moins 12 atomes de carbone ou un reste d'hydrocarbure fluoré ayant au moins 8 atomes de carbone.

6. Procédé selon la revendication 4, caractérisé par le fait que dans la formule générale I, R² est mis pour un reste instable aux bases.
